# EUROPEAN PATENT SPECIFICATION

(11) **EP 2 895 180 B1**
(45) Date of publication and mention of the grant of the patent: **05.04.2017**
(21) Application number: 12772733.7
(22) Date of filing: 14.09.2012
(51) Int. Cl.: A61P 13/02, A61K 36/185, A61K 36/29, A61K 36/756, A61K 36/59, A61K 36/53, A61K 36/718, A61K 36/66

(54) **COMPOSITION FOR THE PREVENTION AND TREATMENT OF ACUTE AND RECURRENT URINARY TRACT INFECTIONS**
ZUSAMMENSETZUNG ZUR PRÄVENTION ODER BEHANDLUNG VON AKUTEN UND REZIDIVIERENDEN HARNWEGSINFEKTIONEN
COMPOSITION POUR LA PRÉVENTION ET LE TRAITEMENT D'INFECTIONS DU TRACTUS URINAIRE AIGUËS ET RÉCURRENTES

(43) Date of publication of application: 22.07.2015
(73) Proprietor: Claride Pharma S.R.L., 20135 Milano (IT)
(72) Inventor: SFERRA, Daniela, I-86093 Carpinone (IT); RUSSO, Valentina, I-86021 Bojano (IT); VERGALITO, Franca, I-86030 Lucito (IT); FRANCESCHINI, Debora, I-86030 Lucito (IT); MANGANO, Katia, I-95128 Catania (IT); SCAPAGNINI, Giovanni, I-95131 Catania (IT); DI MARCO, Roberto, I-95123 Catania (IT); LONGO, Sonia, I-20124 Milano (IT)
(74) Representative: Coppo, Alessandro
(86) International application number: PCT/EP2012/068089
(87) International publication number: WO 2014/040632

(56) References cited:
- WO-A1-2011/000124
- WO-A2-2007/144778
- GEETHA R V ET AL: "Nature's weapon against urinary tract infections", INTERNATIONAL JOURNAL OF DRUG DEVELOPMENT AND RESEARCH 2011 CHAUHAN PUBLISHERS IND, vol. 3, no. 3, July 2011 (2011-07), pages 85-100, XP009164713, ISSN: 0975-9344
- HEAD K A: "Natural approaches to prevention and treatment of infections of the lower urinary tract", ALTERNATIVE MEDICINE REVIEW, THORNE RESEARCH INC., SANDPOINT, US, vol. 13, no. 3, 1 September 2008 (2008-09-01), pages 227-244, XP009148596, ISSN: 1089-5159
- ZDZISINSKA B ET AL: "Therapeutic properties of betulin and betulinic acid, components of birch bark extract", FARMACEUTYCZNY PRZEGLAD NAUKOWY 2010 GRUPA DR A. R. KWIECINSKIEGO POL, vol. 7, no. 3, 2010, pages 33-39, XP009188944, ISSN: 1425-5073

## Description

### Field of the invention

The present invention relates to a composition for the prevention and treatment of acute and recurrent urinary tract infections.

The present invention origins in the field of dietary supplements and herbal drugs. Specifically, the present invention concerns with dietary supplements or herbal drugs for improving of health of the urinary tract or preventing or treating acute and recurrent urinary tract infections (UTI).

### Prior art

A urinary tract infection (UTI) is an infection affecting the kidney, ureter, bladder, or urethra. Most UTIs are caused by gram-negative bacteria and among these the main causal agent is represented by *Escherichia coli,* (∼90% of the cases).

Other bacterial strains such as *Klebsiella, Proteus, Pseudomonas, Enterobacter,* and *Staphlyococcus* and *Serratia,* are also emerging as additional pathogen agents responsible of urinary tract infections.

Urinary tract infection is a condition more common in women than in men. UTI may be asymptomatic but usually is associated with specific symptoms such as urinary frequency, burning pain with voiding, and, in the case where the infection is severe, bleeding with possible presence of pus in the urines.

A single UTI episode affecting the lower urinary tract is very common in the population, especially in adult women, where there is a 50-fold predominance compared with adult men. It has been ascertained that around 97% of female population is affected by at least one case of urinary infection at some point of the live.

Recurrent UTIs is quite common in the female population. It is estimated that it occurs in up to one-third of women after first-episode UTIs. Recurrences requiring intervention are usually defined as two or more episodes over 6 months or three or more episodes over 1 year (this definition applies only to young women with acute uncomplicated UTIs.

Numerous research has led to the finding that most of the urinary infections are caused by the ability of specific bacterial strains such as *E. coli* to firmly adhere to the urothelium.

The urothelium is the epithelial lining of the urinary tract between the renal pelvis and the urinary bladder. Urothelium is composed of at least three layers: a basal cell layer attached to a basement membrane, an intermediate layer, and a superficial or apical layer composed of large hexagonal cells (diameters of 25-250 µm) known as 'umbrella cells'. The urinary tract is one of the major mucosal sites for microbial colonization. The success of *E. coli* as an uropathogen is linked to the expression of type 1 fimbriae. These filamentous appendages enable UPEC both to bind and to invade the superficial urethelial epithelial cells (UECs) lining the bladder lumen. Urinary tract infections with UPEC are initiated by bacterial adherence to uroplakin proteins on the apical surface of umbrella cells. Adherence is followed by bacterial invasion of the bladder wall. Internalization of UPEC in the umbrella cells and formation of intracellular colonies (biofilm-like pods) of UPEC in umbrella cells has been implicated in the mechanism of chronic urinary tract infection. In fact bacteria internalization by urinary epithelial cells could highly benefit the pathogens. In general invasion of host cells by *Escherichia coli* can allow pathogens to evade host defenses and facilitate their dissemination both within and across cellular barriers. Invasion of UECs by UPEC allows these bacteria to avoid the bulk flow of urine trough the bladder and the urinary tract, and give them access to a more nutrient-rich environment. Intracellular uropathogens are also able to avoid the diverse array of antimicrobial substances and immune cells present within the urine and bladder tissue. Furthermore, internalized *Escherichia coli* can persist within the urinary tract virtually undeterred in the face of antibiotic treatments that effectively reduce bacterial titers within the urine. Emerging data indicate that the ability of type 1-piliated *Escherichia coli* strains to invade urinary tract epithelial cells is a key event in the establishment and persistence of a UTI.

The administration of antibiotics represents the standard treatment for UTIs. However, the extended or repeated use of antibiotics could lead to the development of a resistance and to serious side effects.

In addition, there are scientific evidences that the treatment of urinary infections with antibiotics lead to inadequate penetration of bladder epithelial cells, allowing intracellular bacteria to persist.

Eliminating the intracellular pool of bacteria has been shown to have potential clinical benefits, especially reducing recurrence of UTIs. Recent advancements have demonstrated the possibility to induce UPEC exocytosis and elimination in UECs. On the basis of morphological, biochemical and functional properties, it is likely that the intracellular compartments in superficial BECs that harbor UPEC are fusiform vesicles. The exocytosis of fusiform vesicles can be induced by an increase of intracellular cAMP. The activation of this pathway has been identified as possible strategy for treatment of UPEC-infected UECs, in "in vitro" and "in vivo" models, resulting effective in the clearance of UTI.

In view of the above drawbacks caused by UTI treatments with traditional pharmaceutical compounds provided with antibacterial activity, it has been tested the activity on some herbal drugs or supplements.

In the literature it has been reported the ability of cranberry juice and extracts in reducing the symptoms of UTI and in some cases, the recurrence of bacterial infections of the urinary tract.

However, the need of consuming a high amount of juice for obtaining a satisfactory treatment of UTI together with its high sugar and calories content makes difficult a satisfactory treatment with this juice, in particular in diabetic subjects. In addition, the high acidity of cranberry juice often causes stomach upsets in the subjects under treatment.

Geetha R V et al. in the publication "Nature's weapon against urinary tract infections" in the International Journal of drug development and research 2011 Chauhan Publishers Ind., vol. 3 n. 3 July 2011, pages 85-100 disclose that Berberine -as active ingredient of Hydrastis Canadensis- and Arbutin -as active principle of Arctostaphylos uva-ursi- are effective against urinary tract infections. Head K A in "Natural approaches to prevention and treatment of infections of the lower urinary tract" Alternative Medicine Review, Thorne Research Inc., Sandpoint US, vol. 13, n. 3, 1 September 2008 pages 227-244 reports that botanicals that can be effective at the first sign of an infection of the lower urinary tract and for short-term prophylaxis include berberine and *uva ursi.*

The International application WO2007/144778 A2 reports on preventing or treating urinary tract infections involving administering an herb-containing composition comprising Crateva nurvala stem/bark preparation and an Equisetum arvense stem extract preparation and preferably at least one further compound such as Arctostaphylos uva-ursi leaves preparation containing 140 mg arbutin per oral dosage unit.

The International application WO2011/000124 A1 discloses pharmaceutical compositions including viable probiotic strains of Lactobacillus spp. used for the prevention and recovery of individuals with urinary tract infections and immunocompromised individuals.

From Barbara Zdzisinska et al., Therapeutic properties of betulin and betulinic acid, components of birch bark extract, Farm Prezgl Nauk, 2010, 3, 33-39 it is known that the leaves of birch trees are used as diuretic for the treatment of bacterial and inflammatory conditions of urinary tract.

Accordingly at present there is still a need for new remedies suitable for preventing and treating UTI and for alleviating the accompanying symptomatology.

It is a general object of the present invention to overcome some of the above mentioned drawbacks.

It is also an object of the present invention the provision of a composition for preventing recurrence of urinary tract infections.

An additional object of the present invention resides in the provision of a composition for treating infections of the urinary tract caused by more common pathogenic microorganisms in particular uropathogen *Escherichia coli* (UPEC) .

### Summary of the invention

The inventors have discovered that the association of Berberine and Arbutin or vegetal extracts from Berberine and bearberry plants, externalizes an uropathogen microorganism, from infected urothelium. This activity is particularly evident for infections of uropathogen *Escherichia coli.*

The inventors have also founds that the association of plant extracts or parts from plant species containing Berberine and Arbutin inhibits the proliferation of pathogenic bacteria in the urinary tract by reducing their ability from attaching to the epithelial cells of bladder and urethral wall and/or externalizing the uropathogen microorganism from infected urothelium.

By means of such actions, the composition object of the present application is effective in decreasing the incidence of recurrent urinary tract infections, preventing secondary complications and promoting the viability and functions of urethral cells from deterioration, and death related to infective processes.

The present invention thus provides a composition for use in the prevention and treatment of acute and/or recurrent urinary tract infections as claimed in appended claim 1.

In accordance with certain embodiments the composition of the invention contains plant extracts or portions or vegetal tissues of plants containing Arbutin or/and Berberine.

In some embodiments the plant extracts containing Arbutin or/and Berberine are dry vegetal extracts.

The combination of Berberine and Arbutin or of plant extracts containing such vegetal active ingredients provide a synergistic externalization effect on pathogen microorganisms which are responsible of most common infections of the urinary tract.

The composition of the invention is able to inhibit the proliferation of bacterial strains of the urinary tract, especially *Escherichia coli,* by preventing these bacterial strains from attaching to the epithelial cells of bladder and urethral wall. The composition comprises a synergistic association of plant extracts or parts from Berberine, Betulin and Arbutin in an effective amount and an edible carrier for use in the prevention or treatment of UTI.

Typically, the composition of the invention is a pharmaceutical composition or a phytomedicine or a supplement which can be introduced in the dietary regiments of subjects which are susceptible of UTI.

Typically, the synergistic active ingredients of the composition may be of vegetal origin.

The inventors have also found that the effectiveness of the treatment of UTI is further increased by adding Forskolin or a vegetal extract or part from plants containing Forskolin, to the composition containing Berberine, Betulin and Arbutin.

Accordingly, the present invention provides a composition for the prevention and treatment of acute and recurrent urinary tract infections comprising an association of the active ingredients or phytochemical: Berberine, Betulin, Forskolin and Arbutin in a therapeutically effective amount and an edible carrier.

### Brief description of the drawings

Fig. 1 shows urethelial cells 5637 viability, measured by MTT assay, following treatments with different concentrations of single vegetal active ingredients tested on three different solvents.
Figs. 2A, B show urethelial cells 5637 viability, measured by MTT assay, following treatments with different vegetal active ingredients mixtures, in basal condition or after exposure to LPS.
Fig. 3 illustrates *E. coli* adhesion on urothelialcells 5637 in presence of different concentrations of single active ingredients and mixtures of them.
Fig. 4 illustrates *E. coli* internalization (bacterial count) in urothelial cells 5637 in presence of different concentrations of single active ingredients or different active ingredients mixtures.

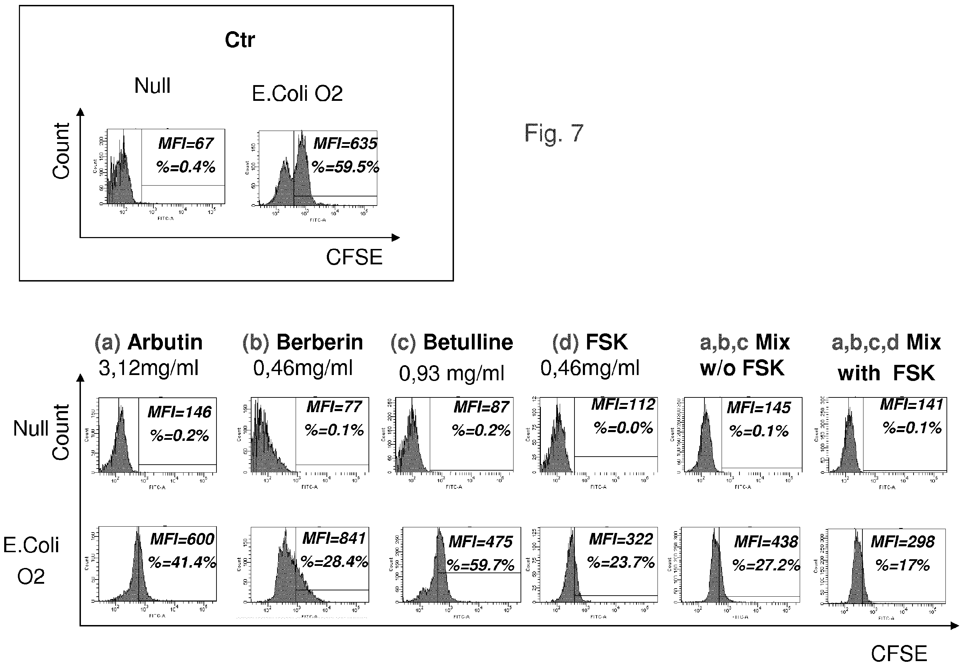

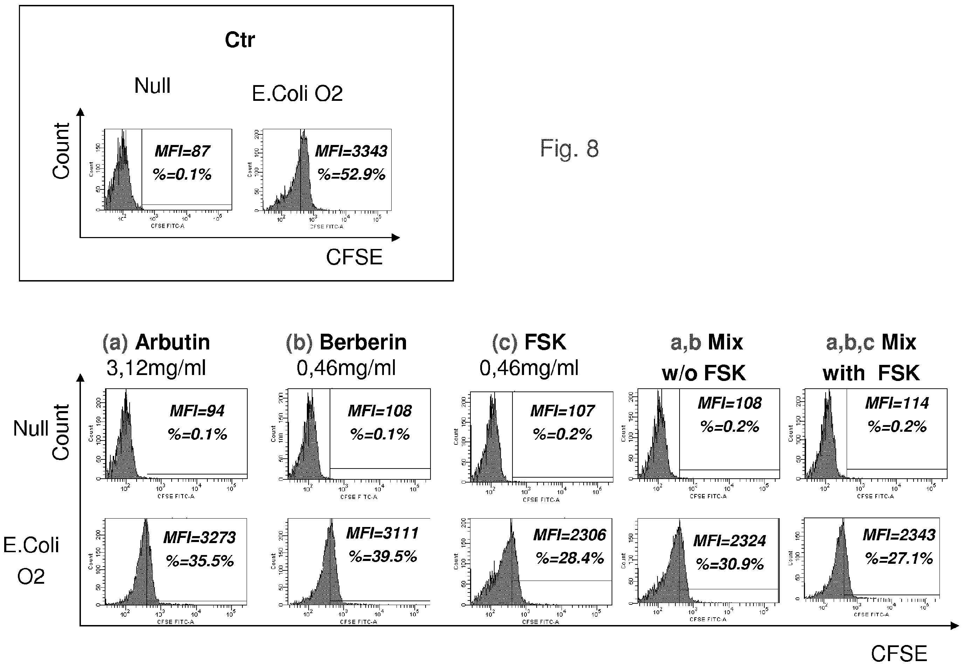

Figs. 5 - 8 show the internalization assay (cytofluorometric method) on urothelial cells 5637 in presence of different concentrations of single vegetal active ingredients or different active ingredients mixtures.

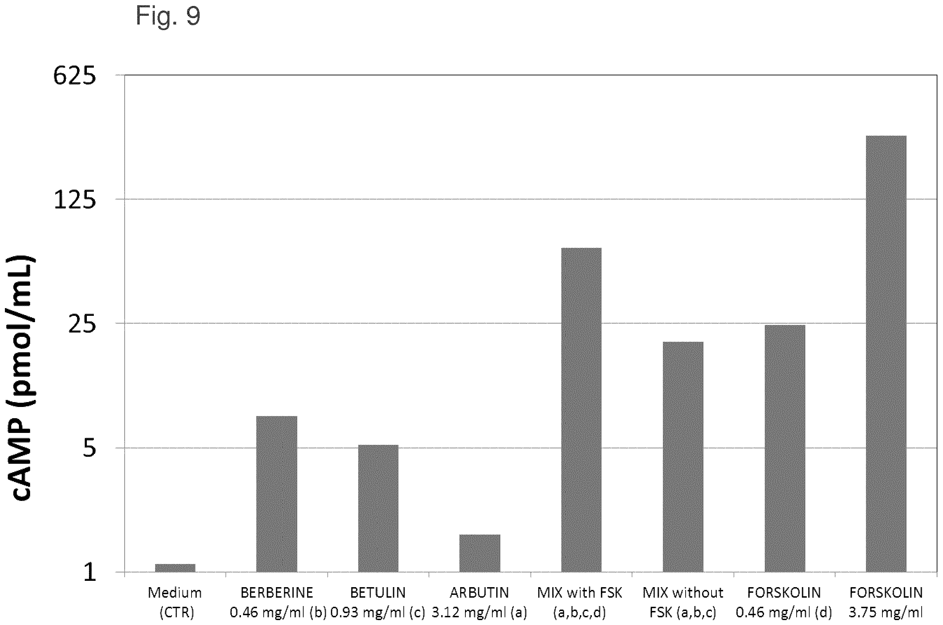

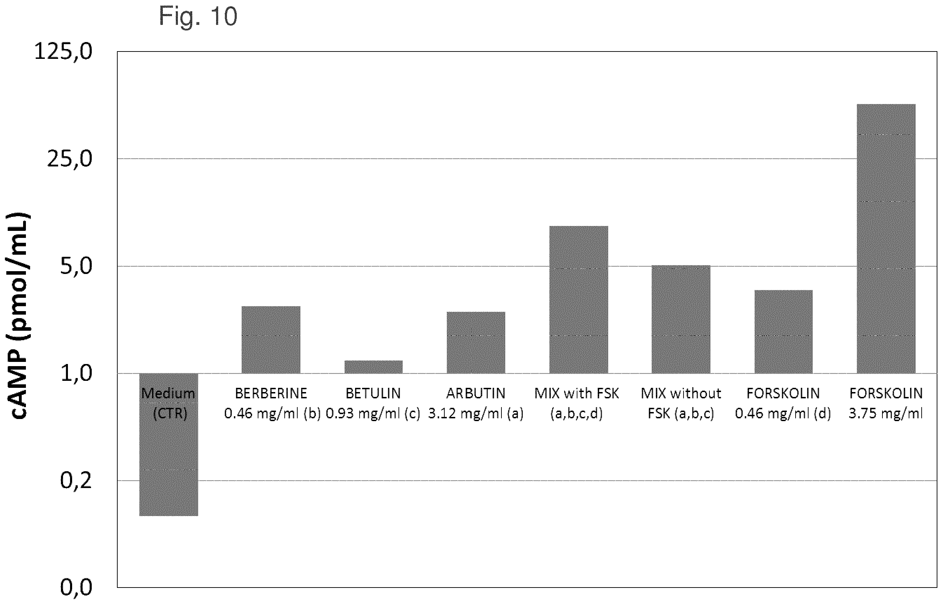

Figs. 9 - 10 show cAMP levels in Urethelial cells 5637 treated with each single vegetal active ingredient or mixtures thereof with or without pre-treatment with the phosphodiesterase inhibitor IBMX, 3-isobutyl-1-methylxanthine, IBMX.

### Detailed description of the invention

In accordance with a first aspect of the present invention, a composition for use as claimed in appended claim 1, is provided.

In accordance with certain aspects of the invention, the inventors found that the association of Berberine and Arbutin reduces the adherence of the bacterial P fimbriae on the urethelial cells surface, thus impairing its colonization and subsequent infection.

One of the active ingredients of the composition of the invention is Arbutin or a vegetal extract or portion or tissue of a plant containing Arbutin.

Arbutin, also referred to as hydroquinone β-D-glucopyranoside [(2R,3S,4S,5R,6S)-2-Hydroxymethyl-6-(4-hydroxyphenoxy)oxane-3,4,5-triol], is a glycosylated hydroquinone extracted from bearberry plant specifically of the genus Arctostaphylos.

It has been reported that Arbutin and its monoester derivatives as disclosed in US Pat. No. 6, 306, 376 which is fully incorporated by reference, inhibits tyrosinase and thus prevents the formation of melanin. For this properties, Arbutin is commonly used as a skin-lightening agent. Arbutin is found in many plants such as members of the Eracaceae, Rosaceae, Saxifragaceae families and specifically in *Bergenia crassifolia.*

The production of Arbutin by chemical synthesis has been reported in the literature, for example in U.S. Patent No. 3,201,385, Us pat. No. 6,388,103. Arbutin can be also produced by common extraction techniques from plants and parts thereof naturally containing Arbutin, or by bio-transformation using either microbial hosts or plant cell cultures, seeds, or seedlings expressing suitable glucosyltransferases contacted with hydroquinone and UDP-glucose (Arend et al.; Phytochemistry 53:187-193 -2000).

Arbutin founds application generically, in the depigmentation of the skin. In the composition of the invention Arbutin synergistically contributes to the prevention and/or treatment of UTI, in particular caused by urothelium colonization by *E. coli.* In certain embodiments, Arbutin or a plant extract thereof may be present in the composition in an amount of 0,01 to 90% by weight, preferably of 20 to 65% by weight.

The association of Arbutin and Berberine, with Betulin and optionally Forskolin, synergistically prevents the recurrence of UTIs, either activating exocytosis and elimination of UPEC, via the activation of cAMP pathway in infected UEC or counteracting the adhesion rates of *E. coli* to the uroepithelial cells.

Berberine or a vegetal extract or portion or tissue of a plant containing Berberine is one of the active ingredients of the composition of the invention.

Berberine is a plant alkaloid which may be found in the roots, rhizomes or stem bark of Berberine-containing plants such as *Berberis vulgaris* (barberry), *Berberis aristata* (tree turmeric), *Berberis* (e.g. *Berberis aquifolium* (Oregon grape), *Hydrastis canadensis* (Goldenseal), *Coptis chinensis* (Chinese Goldthread, Coptis), *Phellodendron amurense* (Amur Cork Tree, Huang Bai, Huang Po, Po Mu) *Tinospora cordifolia,* or, in smaller amounts also in *Eschscholzia californica* (Californian Poppy) or *Argemone mexicana* (Prickly Poppy).

Typically, Berberine is a quaternary ammonium salt from the protoberberine group of isoquinoline alkaloids.

In certain embodiments the Berberine alkaloid can obtained from *Berberis* extracts, decoctions or parts of plants containing such alkaloid.

In certain embodiments of the invention Berberine is obtained as a dry extract from a portion of a plant containing the alkaloid, *Berberis Aristata,* especially.

In certain embodiments, Berberine or a plant extract thereof may be present in the composition in an amount of 0,01 to 35% by weight, preferably of 0,5 to 20% by weight.

In certain embodiments, Betulin or a vegetal extract or portion or tissue of a plant containing betulin is an active ingredient of the composition of the invention.

Betulin is a naturally-occurring pentacyclic triterpene alcohol of the lupine family, also referred as betulinol or lup-20(29)-ene-3β,28-diol. It is commonly isolated from the bark of some tree species, especially in the birch (*Betula sp.*) bark in amounts up to 40% of the bark dry weight. Betulin can also be obtained from extracts, decoctions or plant parts of Betula species.

In the field of plant extraction methods are known for the isolation of betulin from bark material, typically based on extraction.

Betulin can be converted to betulinic acid which is the alcohol group replaced by a carboxylic acid group, which is biologically active itself.

Betulinic acid may be isolated for example from Betula sp. (birch) barch or cork or cork oak (*Quercus suber L.*) by extraction. In addition, betulinic acid may be produced by alternative methods such as those disclosed in US patent 5,804,575 or US patent 6,280,778.

In certain embodiments, betulinic acid and or betulin or a plant extract thereof may be present in the composition in an amount of 0,01 to 35% by weight, preferably of 0,5 to 10% by weight.

In certain embodiments, the composition of the present invention comprises a therapeutically effective amount of Arbutin, Berberine and Betulin or betulinic acid, or vegetal extracts thereof, and an edible carrier.

The inventors have found that the prophylactic or therapeutic activity of a composition containing Arbutin and Berberine is increased when at least an additional active ingredient of vegetal origin selected from betulin, betulinic acid and Forskolin is added.

In certain embodiments the composition of the invention further comprises Forskolin or a vegetal extract or portion or tissue of a plant containing Forskolin as additional active ingredient.

Forskolin is a diterpenoid compound described, for example in U.S. Pat. No. 4,088,659. Typically, Forskolin, originates from the Asiatic herb *Coleus Forskohlii.*

Typically, Forskolin may be obtained by conventional extraction techniques from known plants such as *Coleus Forskohlii.* Herbal preparations from *Coleous Forskohlii* found applications in the medical filed for example in the treatment of allergic conditions, asthma, psoriasis, cardiovascular disorders, and ophthalmic conditions such as lowering internal eye pressure and glaucoma prevention. Forskolin has been also used in the management of obesity, in digestive and malabsorption syndromes. It is reported in the scientific literature that Forskolin acts by activating and stimulating adenylate cyclase, an enzyme regulating the formation of cyclic AMP, a substance which exerts a control on some cell activities. It has been also reported that Forskolin also acts by inhibiting membrane transport proteins and cannel proteins through mechanism of action not involving the activation of cAMP.

The moderate toxicity of Forskolin observed at high doses has limited its use alone on a large scale.

In certain embodiments, Forskolin or a plant extract containing it may be present in the composition in an amount of 0,01 to 35% by weight, preferably of 0,5 to 10% by weight.

The inventors have now also found that combining Forskolin with Berberine, Betulin and Arbutin or vegetal extracts thereof, a synergistic increase cAMP levels is obtained which result in beneficial effect on the prevention and/or treatment of recurrent UTIs.

The composition of the invention comprises Betulin as active ingredient in addition to Berberine and Arbutin or its aglycone hydroquinone.

According to certain embodiments the Applicant has designed an experimental model to determine the synergistic therapeutical effect of Berberine and Arbutin, together with Betulin, in preventing urothelium infection by uropathogen such us *E*. *coli,* and/or recurrent UTI. This is because the combination of the above active ingredients or extracts containing them, activate a pathway of cell response to infection and facilitate expulsion and cleavage of the uropathogen from uroepithelia.

More in detail, the above combination induce and over express intracellular levels of cAMP. Typically, cAMP upregulation induces exocytosis of fusiform vesicles from urethelial cells, resulting in externalization and reduction number of intracellular *E. coli.*

Typically, the composition of the invention exerts a preventive effect, on the middle/long term of systematic intake, in retarding, preventing and partially treating urinary tract infections (UTI) in particular caused by uropathogen *Escherichia coli* (UPEC).

In certain embodiments one or more of the above disclosed active ingredients of the composition of the invention are provided in the form of dry vegetal extracts.

A composition according to the present invention may optionally contain other active ingredients.

Typically, the inclusion of a probiotic in the formulation of the composition of the invention enhances the capability of preventing and/or treating recurrent cystitis.

In accordance with certain embodiments the composition thus further contains a probiotic, as additional active ingredient, such as a Lactobacillus acidophilus.

A suitable probiotic is Danisco's probiotic strains *Lactobacillus acidophilus* NCFM®.

In certain embodiments the composition of the invention further includes one or more vitamins, such as vitamin of the B group, niacin, vitamin A, vitamin C, vitamin PP and others.

In accordance with some embodiments the composition of the invention also includes one or micro micronutrients and/or minerals or micronutrients such as salts of potassium, sodium, Zn, Fe, Cr, Se, Mn, Mg and others.

The term "carrier" refers to a vehicle, excipient, diluent, or adjuvant with which the association of therapeutic or active ingredients is administered. Any carrier and/or excipient suitable for the form of preparation desired for administration is contemplated for use with the compounds disclosed herein.

For purposes of the present application, the term "edible" is intended to mean food grade materials which are approved by regulatory authorities for use in pharmaceutical or food applications.

The compositions of the present invention encompass any compositions made by admixing the association of active ingredients of vegetal origins of the present invention and a pharmaceutically acceptable carrier. Such compositions are suitable for nutritional, pharmaceutical or dietetic use in an animal or human.

The carrier may take a wide variety of forms depending on the form of preparation desired for the oral administration, e.g., tablets, capsules, powders. In preparing the compositions for oral dosage form, any of the usual nutritional or pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

In certain embodiments, the compounds of the present invention can be combined as the active ingredient in intimate admixture with a suitable edible carrier and/or excipient according to conventional pharmaceutical/nutritional techniques or food processing.

An exemplary route of administration of the composition of the invention is the oral route. The compositions may be conveniently presented in unit dosage form and prepared by any of the methods well-known in the art of pharmacy or food processing.

The pharmaceutical compositions may be in the form of tablets, pills, capsules, solutions, suspensions, emulsion, powders, and as sustained release formulations.

If desired, tablets may be coated by standard aqueous or non-aqueous techniques. In certain embodiments such compositions and preparations can contain at least 0.1 percent of each of the active compound. The percentage of active compound in these compositions may, of course, be varied and may conveniently be between about 1 percent to about 60 percent of the weight of the unit. The amount of active compound in such compositions is such that a prophylactic or therapeutically effective dosage will be obtained.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

In certain embodiments, the composition of the invention further includes one or more additional components, such as additives, fillers, stabilizers, emulsifying, texturizing, filmogenic, plasticizing, wetting agents, and thickeners.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor. To prevent breakdown during transit through the upper portion of the gastrointestinal tract, the composition may be an enteric coated formulation.

In some embodiments, in the pharmaceutical compositions of the present invention the active principle or active principles are generally formulated in dosage units. The dosage unit may contain from 0.1 to 1000 mg of active compound per dosage unit for daily administration.

In some embodiments, the amounts effective for formulation will depend on the severity of the disease, disorder or condition, previous therapy, the individual's health status and response to the association of active ingredients. In some embodiments, the dose is in the range from 0.001% by weight to about 60% by weight of the formulation.

In accordance with certain embodiments the composition of the invention is a herbal drugs that is product containing plant materials or plant parts as active ingredients in the crude or processed state and ore or more carriers.

In accordance with certain embodiments the composition of the invention is a supplement or nutritional product.

With the term of "synergism or synergistic activity" is meant an activity which is greater than the sum of the activities of the active ingredient when taken alone.

Drug synergism occurs when two active ingredient interact in ways that enhance or magnify one or more effects, or side effects, of them. In other words, two active ingredients that produce overtly similar effects will sometimes produce exaggerated or diminished effects when used concurrently, and a quantitative assessment is necessary to distinguish these cases from simply additive action (Tallarida RJ. Drug synergism: its detection and applications. J Pharmacol Exp Ther. 2001 Sep;298(3):865-72). The biological effects on which we are focusing are positive modulation of cAMP in UEC and consequent induction of UPEC exocytosis and elimination. The pharmacological definition of synergism, when translated in terms of molecular events, relates often to the ability of the two bioactive substances producing synergism to enhance cell functions by eliciting pathways which are originally distinct but converge at the higher levels in elementary cell activities concurring to the same major cell program. In other words, if there are two ways for a cell to avoid death controlled by two unrelated or minimally related pathways A and B, enhancement of only A or B by two compounds is likely to produce at the highest doses saturation of the pathway, and therefore a combined effect which is less than additive. Full stimulation of A and B, instead, could produce additivity or even synergism (a) because all the activities bringing to that response are engaged, (b) because the cross-talks taking place where the pathways converge can become mutually enhancing, or, finally, (c) because the synchronous activation of both allow the cell to move into discrete but sequential steps of activity.

The following examples are provided merely for illustrating the present invention.

### Example 1

Synergistic composition for preventing or treating urinary tract infections with the following formulation:

| Vegetal active ingredient (Plant extract) | Stock Sol | Solvent | work solution |
|---|---|---|---|
| Berberine | 3mg/mL | H2O | 1:20 |
| Betulin | 50mg/mL | H2O:DMSO (80:20) | 1:10 |
| Arbutin | 50mg/mL | H2O | 1:10 |
| Forskolin (*Coleus forskohlii*) | 50mg/mL | H2O:DMSO (80:20) | 1:10 |
| Plants mix Berberine-Betulin-Arbutin (v/v/v) | 50mg/mL | H2O:DMSO (80:20) | 1:10 |

### Example 2

### Antimicrobial proprieties

To further characterize the mechanism of bacterial invasion through fusiform vesicles, we studied a type 1 fimbriated *E. coli* strain, ORN103(pSH2)11, and the human 5637 BEC line (HTB-9, ATCC).

### Micro-organisms tested

The following bacterial were tested for their susceptibility:
Lactobacillus acidophilus
*E. coli* O1
*E. coli* 02
*E. coli* O11
*E. coli* O55
*Enterococcus faecalis*
*Proteus vulgaris*

The micro-organisms were grown overnight at 37°C in Mueller-Hinton Broth (Oxoid, England) at pH 7.4.

### Antibacterial testing

Antimicrobial activity of the crude extracts of different plants was determined by the liquid micro dilution method (van den Berge & Vlietinick 1991).

Seven 2-fold dilutions of the plant-extract, sterilized by filtration through a 0.23 µm membrane filter, were carried out starting from the dilution indicated in table 1. The tubes with the substances and broth were inoculated with a micro-organism suspension at a density of 10⁵CFU per ml. The tubes were incubated at 37°C for 24 h. and then observed for determine the Minimum Inhibitory Concentration (MIC). The growth of organisms was observed macroscopically as medium turbidity and further determined by a spectrophotometer (Victor2-Perkin Elmer) at 620 nm. Control wells containing germs without extracts or vice versa extracts without bacteria were assayed simultaneously. All conditions were tested in triplicates.

### Cell lines

Human tumor cells bladder were grown in RPMI medium supplemented with 10% FBS, penicillin and streptomycin and L-glutamine on tissue culture flats, in 5% CO in air into 24-well plates and grown to confluence.

### Labelling of bacteria

Bacteria grown overnight were pelleted, washed and resuspended in sterile PBS to a final concentration of 10 E6 cfu/ml and then labelled with CFDA-SE, 5 mM at RT for 20 min. Fluorescent labelling was terminated by pelleting bacteria and washing once in PBS to remove excess CFDA-SE. Bacteria were gently resuspended in 1 ml of medium and co-incubated with cells.

### Internalization assay

Labelled bacteria were co-incubated wit the e bladder cell for 2 h at 37°C. Coltures were then washed twice with RPMI and then incubated for other 2 h in medium containing 100 µg/ml membrane-impermeable bactericidal antibiotic ampicillin with plants at various concentrations. Cells were then washed three times with PBS, detached manually with a scraper and finally analyzed by the flow cytometer.

### Flow cytometry measurement

The fluorescent intensity of the bladder cells with internalized bacteria was measured with FACSCanto equipment (Becton Dickinson). The data obtained from the flow cytometer were analyzed using FACS Diva software (Becton Dickinson).

The initial analysis of the cells was carried out in the dot plot display mode of forward scatter (FCS) versus side scatter (SSC). FCS in the x-axis is a measure of the size of the detected particle and SSC in the y-axis measures the cellular granularity. Based on the FCS v. SSC analysis of the cells and staining of the cells with the FITC conjugated the flow cytometer was set to include bladder cells and to gate out cellular debris and non-adherent bacteria. The numerical results where expressed either as geometric mean fluorescent intensity (MFI) values of a cell population.

### Measurement of bacterial viability

In order to confirm that internalized cells retain their vitality, an aliquot of 500µl from each well (treated as described above) was washed three times with PBS, lysed in 1 ml of 0.1 % Triton X-100 in ddH2O, plated on Muller Hinton agar dishes and incubated over night at 37°C. Colonies were then manually counted 18h later.

### Cyclic AMP detection

Human tumor cells bladder were seeded into 96 wells plate and incubated in complete RPMI (with 10% FCS) to allow the adhesion and growth to confluence.

Cells were treated similarly as internalization assay described above. Cyclic AMP was detected by immunoenzymatic ELISA kit according the supplier instruction (Arbor Assay, Michigan, USA). Briefly cells were washed with PBS and the Sample Diluent was directly added for 10 minutes at room temperature. Cells were dislodged from the plate surface, inspected to ensure lysis and further centrifuged at ≥600 x g at 4°C for 15 minutes. The supernatant were directly assayed following the protocol supplied by the ELISA kit.

### Example 3

The uroepithelial Cell line ATCC 5637 was exposed to the test compounds in a dose range between 25 and 12:39 mg / ml, in order to exclude of a direct toxicity In vitro of the extracts,

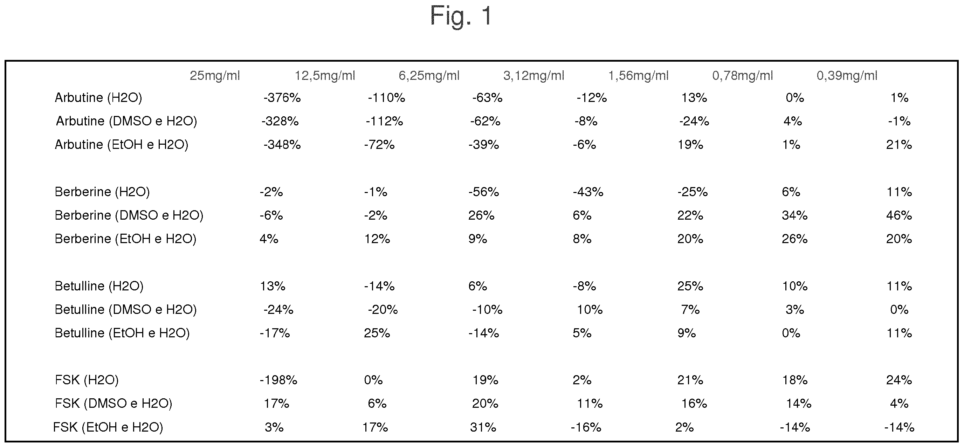

During this series of experiments (Fig. 1) the phytochemical's dry powders were solubilized with different solvents:
a) 100% water (H₂O);
b) 20% of DMSO and then further diluted with 80% of H₂O
c) 20% of ethanol (EtOH) and then further diluted with 80% of H₂O.

No evident signs of toxicity were evident for doses equal or lower than 3.12 mg/ml for Arbutin and Berberine, 12,5 mg/ml for Berberine and Forskolin and 6.25mg/ml for Betulin and 3.12 mg/ml.

Despite were not revealed significant differences between the three solvents tested the subsequent experiments were conducted solubilizing the phytochemicals on DMSO/H₂ mix (20/80-v/v).

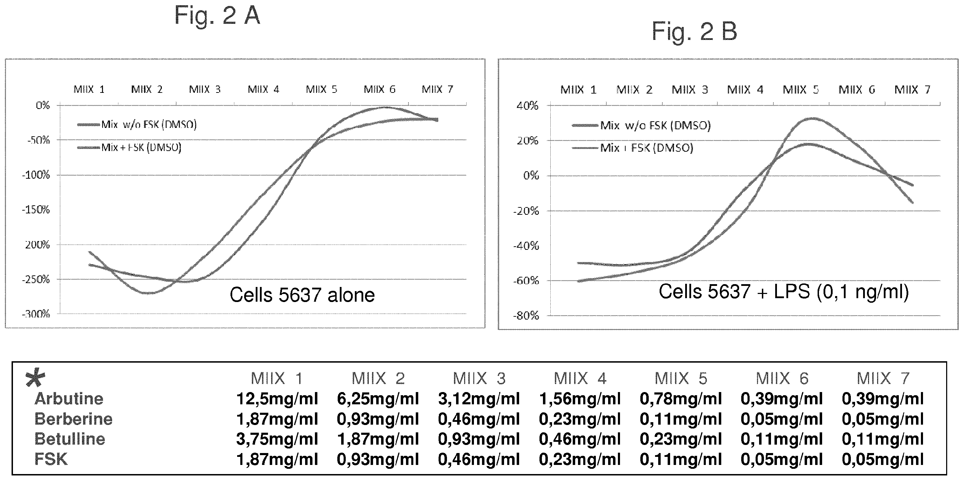

Based on previous results the extracts were mixed and tested (Fig. 2) on a variable ratio as follows: Arbutin (55-65%), Berberine (9-11%), Betulin (18-20%), Forskolin (9-11%). In order to verify that phytochemicals when mixed do not exhibit synergistic toxicity the same tests, as described on Fig. 1, was repeated exposing the cells to the mix of plants.

The same test was also conducted in presence of 0.1 ng/ml of LPS (Fig. 2b), circumstance that may resemble in vitro the interaction between the uroepithelia and the gram negative bacteria. in both conditions, except for the three higher doses, no sign of relevant toxicity was detectable.

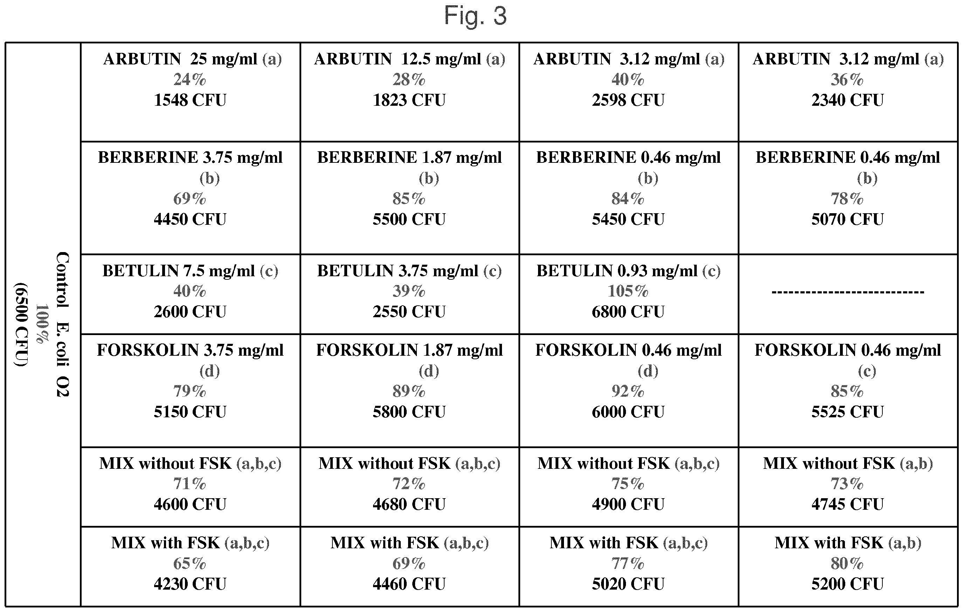

The ability of the phytochemicals to interfere with *E. coli* adhesion to the cellular surface was tested (Fig. 3). Test substances were assayed alone and in combination according to the same proportion described above. The number of colony-forming unit (CFU) in medium alone was assumed as 100%. Betulin, Berberin and Forskolin, when tested alone, did not exert more than a moderate reduction in adherent bacteria count.

Vice versa Arbutin alone was capable of reducing counts to 40% even at low dose (3.12 mg/ml).

Likewise the mix of substances, albeit to a lesser extent, counteracts the *E*. *coli* adhesion. In addition the effect was detectable in both mixtures suggesting that this effect was independent from the presence of Forskolin.

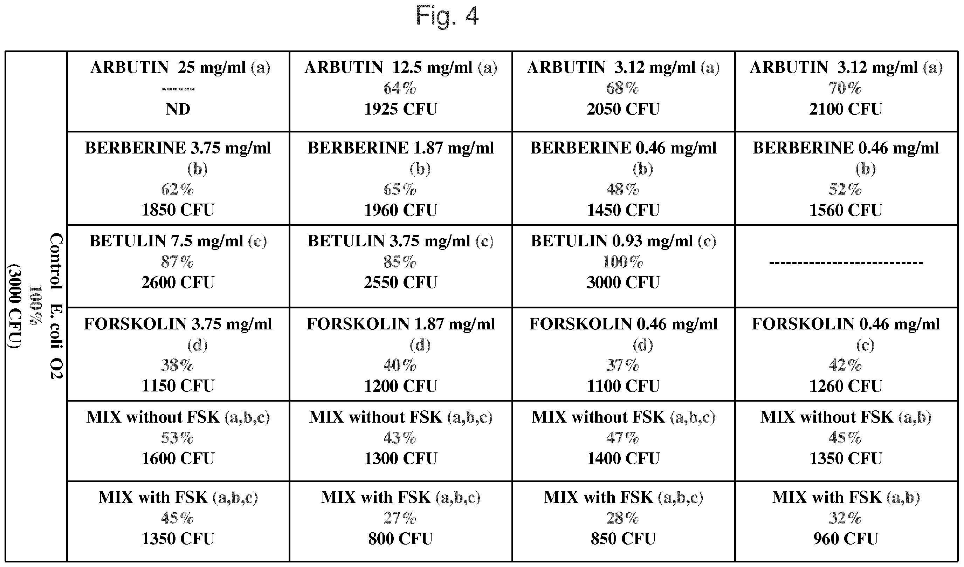

A "canonical" internalization assay (Fig. 4) was performed aim to assess the ability of the test substances (alone or in combination) in inducing cells 5637 to extrude *E.coli* previously internalized.

As expected, Forskolin was capable of reducing bacterial counts of bacteria at all the three doses tested. In comparison either Arbutin or Berberine were partially active even if in a lesser extent.

Notables the results obtained with the mix of plants.

Both formulas (with or without Forskolin), when compared either to the medium alone or to the single components, showed remarkable synergistic effects on promoting the bacterial extrusion.

Respectively, was obtained a reduction of CFU equal to about the 50% with the mix without FSK and as to 80% on the complete formula.

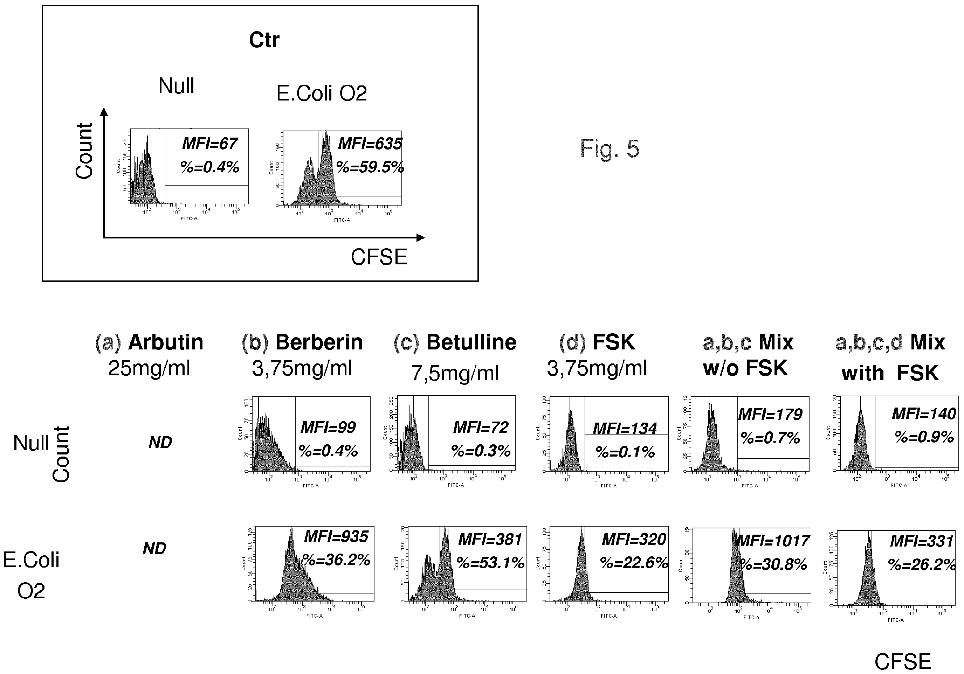

Experiments were repeated (Fig.5a, b, c.) with a different methodology in order to confirm results obtained from the internalization assay performed with microbiological count supplementary. *E. coli,* pre stained with carbossi-fluorescein, were let to adhere and internalize to 5737 cells. Cultures, treated as described previously and detailed in M&M, were then analyzed in cytofluorimetry. As expected were obtained similar and comparable results to the microbiological ones. Once again the treatment with plant mixture was able to contrast and reduce the internalization's process of *E. coli.* Furthermore, this was evident when Forskolin was present but even the lack of this component in to the formula only partially reduced the potency of the effects.

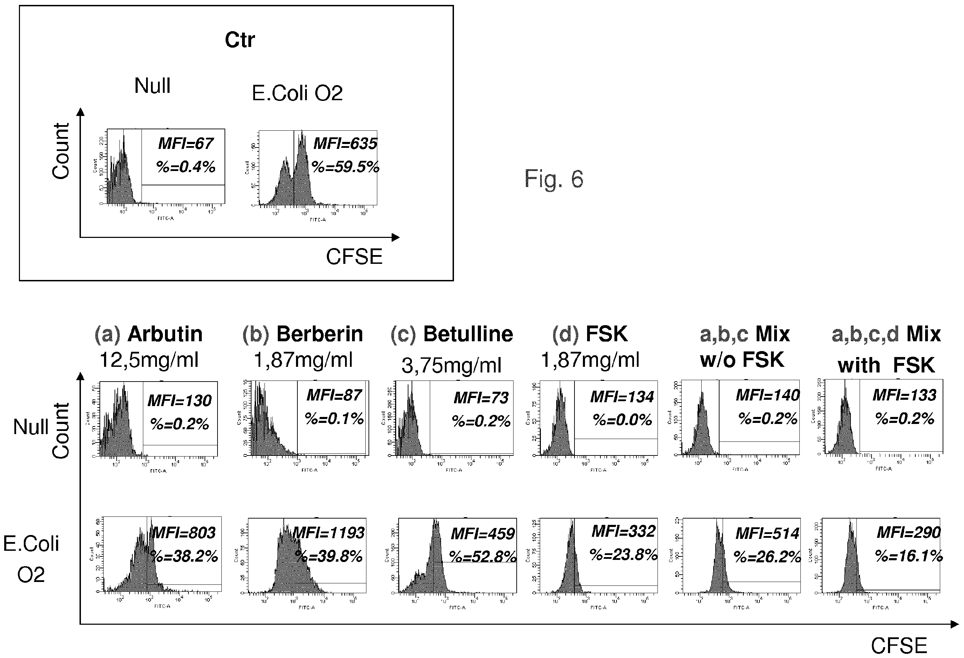

Forskolin is commonly used to raise levels of cyclic AMP (cAMP) which increasing production drives the extrusion process of bacteria from urothelial cell. (Fig 6 a, b) With a view to ascertain a possible mechanism of action the cAMP levels were measured in coltures of 5637 cell previously exposed to different doses of phytochemicals alone or in combination.

The discovery that cAMP regulates the intracellular niche of UPEC has revealed a new and potentially effective strategy for combating UTI. The compositions that increase cAMP within UECs speed the time-to-resolution of UTI and decrease morbidity. Pharmacotherapies that increase intracellular cAMP, combined with traditional antibiotics, may lead to large clinical benefits in refractory and recurrent UTIs.

### Example 4

A composition for use in the prevention and treatment of recurrent urinary tract infections having the following formulation
Berberine 75 mg
Forskolin (Coleous) 30 mg
Arbutin Extract 330 mg
Betulin Extract 55 mg
Probiotic 20 mg

### Example 5

A composition for use in the prevention and treatment of recurrent urinary tract infections having the following formulation
Berberine Extract 75 mg
Arbutin Extract 330 mg
Betulin Extract 55 mg
Probiotic 20 mg

## Claims

1. A composition for use in the prevention and treatment of acute and/or recurrent urinary tract infections comprising a synergistic combination of Berberine, Betulin and Arbutin in an effective amount and an edible carrier.

2. A composition for use according to claim 1 wherein Berberine is in the form of a vegetal extract or portion of a plant containing Berberine.

3. A composition for use according to claim 2 wherein the plant containing Berberine is selected from *Berberis, Coptis chinensis, Phellodendron amurense, Tinospora cordifolia, Eschscholzia californica, Argemone Mexicana* and mixtures thereof.

4. A composition for use according to claim 1 wherein Arbutin is in the form of a vegetal extract or portion of a plant containing Arbutin.

5. A composition for use according to claim 4 wherein the plant containing Arbutin is bearberry plant.

6. A composition for use according to claim 1 wherein betulin is in the form of a vegetal extract or portion of a plant containing betulin or betulinic acid.

7. A composition for use according to claim 6 wherein the plant containing betulin belongs to the *Betula species* or cork or cork oak.

8. A composition for use according to anyone of claims 1 to 7 further comprising Forskolin as vegetal active ingredient.

9. A composition for use according to claim 8 wherein Forskolin is in the form of a vegetal extract or portion of a plant containing Forskolin.

10. A composition for use according to claim 9 wherein the plant containing Forskolin is *Coleus Forskohlii.*

11. A composition for use according to anyone of claims 1 to 10 comprising Arbutin, Berberine, Forskolin, betulin and/or betulinic acid or vegetal extracts and/or plant portions and/or vegetal tissues containing them as active ingredients and an edible carrier.

12. A composition for use according to anyone of claims 1 to 11 further comprising a probiotic and or at least one ingredients selected from vitamins, minerals, micronutrients and mixtures thereof.

13. A composition for use according to claim 12 wherein the probiotic is a Lactobacillus acidophilus.

14. A composition for use according to anyone of claims 1 to 13 in the form of a tablet or of a powder optionally encapsulated.

15. A composition for use according to anyone of claims 1 to 14 wherein the recurrent urinary tract infection is caused by gram-negative bacterium.

16. A composition for the use according to claim 15 wherein the gram-negative bacterium is *Escherichia coli.*

## Patentansprüche

1. Zusammensetzung zur Verwendung zur Prävention und Behandlung von akuten und/oder wiederkehrenden Harntraktinfektionen, umfassend eine synergistische Kombination aus Berberin, Betulin und Arbutin in einer wirksamen Menge und einem essbaren Träger.

2. Zusammensetzung zur Verwendung nach Anspruch 1, wobei Berberin in der Form eines pflanzlichen Extrakts oder Teils einer Berberin enthaltenden Pflanze vorliegt.

3. Zusammensetzung zur Verwendung nach Anspruch 2, wobei die Berberin enthaltende Pflanze aus *Berberis, Coptis chinensis, Phellodendron amurense, Tinospora cordifolia, Eschscholzia californica, Argemone mexicana* und deren Mischungen ausgewählt ist.

4. Zusammensetzung zur Verwendung nach Anspruch 1, wobei Arbutin in der Form eines pflanzlichen Extrakts oder Teils einer Arbutin enthaltenden Pflanze vorliegt.

5. Zusammensetzung zur Verwendung nach Anspruch 4, wobei die Arbutin enthaltende Pflanze Beerentraubenpflanze ist.

6. Zusammensetzung zur Verwendung nach Anspruch 1, wobei Betulin in der Form eines pflanzlichen Extrakts oder Teils einer Betulin oder Betulinsäure enthaltenden Pflanze vorliegt.

7. Zusammensetzung zur Verwendung nach Anspruch 6, wobei die Betulin enthaltende Pflanze zu den *Betula-*Spezies oder Kork oder Korkeiche gehört.

8. Zusammensetzung zur Verwendung nach einem der Ansprüche 1 bis 7, die ferner Forskolin als pflanzlichen aktiven Bestandteil umfasst.

9. Zusammensetzung zur Verwendung nach Anspruch 8, wobei Forskolin in der Form eines pflanzlichen Extrakts oder Teils einer Forskolinen enthaltenden Pflanze vorliegt.

10. Zusammensetzung zur Verwendung nach Anspruch 9, wobei die Forskolin enthaltende Pflanze *Coleus forskohlii* ist.

11. Zusammensetzung zur Verwendung nach einem der Ansprüche 1 bis 10, die Arbutin, Berberin, Forskolin, Betulin und/oder Betulinsäure oder diese enthaltende pflanzliche Extrakte und/oder Pflanzenteile und/oder pflanzliche Gewebe als aktive Bestandteile und einen essbaren Träger umfasst.

12. Zusammensetzung zur Verwendung nach einem der Ansprüche 1 bis 11, die ferner ein Probiotikum und/oder zumindest einen Bestandteil umfasst, der aus Vitaminen, Mineralien, Mikronährstoffen und deren Mischungen ausgewählt ist.

13. Zusammensetzung zur Verwendung nach Anspruch 12, wobei das Probiotikum Lactobacillus acidophilus ist.

14. Zusammensetzung zur Verwendung nach einem der Ansprüche 1 bis 13 in der Form einer Tablette oder eines Pulvers, optional verkapselt, ist.

15. Zusammensetzung zur Verwendung nach einem der Ansprüche 1 bis 14, wobei die wiederkehrende Harntraktinfektion durch ein gramnegatives Bakterium hervorgerufen wird.

16. Zusammensetzung zur Verwendung nach Anspruch 15, wobei das gram-negative Bakterium *Escherichia coli ist.*

## Revendications

1. Composition pour une utilisation dans la prévention et le traitement d'infections aiguës et/ou récurrentes des voies urinaires comprenant une combinaison synergique de berbérine, de bétuline et d'arbutine dans une quantité efficace et un support comestible.

2. Composition pour une utilisation selon la revendication 1, dans laquelle la berbérine est sous la forme d'un extrait végétal ou d'une partie d'une plante contenant de la berbérine.

3. Composition pour une utilisation selon la revendication 2, dans laquelle la plante contenant de la berbérine est choisie parmi *Berberis, Coptis chinensis, Phellodendron amurense, Tinospora cordifolia, Eschscholzia californica, Argemone Mexicana* et leurs mélanges.

4. Composition pour une utilisation selon la revendication 1, dans laquelle l'arbutine est sous la forme d'un extrait végétal ou d'une partie d'une plante contenant de l'arbutine.

5. Composition pour une utilisation selon la revendication 4, dans laquelle la plante contenant de l'arbutine est la plante de busserole.

6. Composition pour une utilisation selon la revendication 1, dans laquelle la bétuline est sous la forme d'un extrait végétal ou d'une partie d'une plante contenant de la bétuline ou de l'acide bétulinique.

7. Composition pour une utilisation selon la revendication 6, dans laquelle la plante contenant de la bétuline appartient à l'espèce *Betula* ou au liège ou chêne-liège.

8. Composition pour une utilisation selon l'une quelconque des revendications 1 à 7 comprenant en outre de la forskoline en tant que principe actif végétal.

9. Composition pour une utilisation selon la revendication 8, dans laquelle la forskoline est sous la forme d'un extrait végétal ou d'une partie d'une plante contenant de la forskoline.

10. Composition pour une utilisation selon la revendication 9, dans laquelle la plante contenant de la forskoline est *Coleus Forskohlii.*

11. Composition pour une utilisation selon l'une quelconque des revendications 1 à 10 comprenant de l'arbutine, de la berbérine, de la forskoline, de la bétuline et/ou de l'acide bétulinique ou des extraits végétaux et/ou des parties de plantes et/ou des tissus végétaux les contenant en tant que principes actifs et un support comestible.

12. Composition pour une utilisation selon l'une quelconque des revendications 1 à 11 comprenant en outre un probiotique et/ou au moins un principe choisi parmi des vitamines, des minéraux, des micronutriments et leurs mélanges.

13. Composition pour une utilisation selon la revendication 12, dans laquelle le probiotique est un Lactobacillus acidophilus.

14. Composition pour une utilisation selon l'une quelconque des revendications 1 à 13 sous la forme d'un comprimé ou d'une poudre éventuellement encapsulée.

15. Composition pour une utilisation selon l'une quelconque des revendications 1 à 14, dans laquelle l'infection récurrente des voies urinaires est provoquée par une bactérie Gram négatif.

16. Composition pour l'utilisation selon la revendication 15, dans laquelle la bactérie Gram négatif est *Escherichia coli.*
